(19) Europäisches Patentamt
European Patent Office
Office européen des brevets

(11) **EP 3 147 037 A2**

(12) **EUROPEAN PATENT APPLICATION**

(43) Date of publication:
**29.03.2017 Bulletin 2017/13**

(51) Int Cl.:
*B06B 1/02* (2006.01)     *A61B 8/08* (2006.01)
*A61B 8/00* (2006.01)     *A61B 5/00* (2006.01)
*G01N 29/24* (2006.01)

(21) Application number: **16187110.8**

(22) Date of filing: **02.09.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **04.09.2015 JP 2015175022
24.06.2016 JP 2016125590**

(71) Applicant: **Canon Kabushiki Kaisha
Tokyo 146-8501 (JP)**

(72) Inventors:
• **TANAKA, Takatoshi**
  **Ohta-ku, Tokyo 146-8501 (JP)**
• **NAKABAYASHI, Takaaki**
  **Ohta-ku, Tokyo 146-8501 (JP)**
• **ITO, Nobuhiro**
  **Ohta-ku, Tokyo 146-8501 (JP)**

(74) Representative: **Houle, Timothy James
  Canon Europe Ltd
  European Patent Department
  3 The Square
  Stockley Park
  Uxbridge, Middlesex UB11 1ET (GB)**

(54) **TRANSDUCER ARRAY, AND ACOUSTIC WAVE MEASUREMENT APPARATUS**

(57)     A transducer array (32) including a support member (322), an inner surface of which is defined by a quadric surface of revolution (45) formed by rotating an imaginary quadratic curve (41, 42, 43, 44) about an imaginary rotation axis (46), the inner surface provided with a plurality of openings (40), and a plurality of transducers (321) including receiving surfaces (10) having a substantially parallelogram shape so as to confirm with the openings. In the receiving surface, one vertex among four vertexes (vr1, vr2, vr3, vr4) is proximate with the imaginary rotation axis than the other three vertexes.

FIG. 1A

## FIG. 1B

32

LEFT-HANDED HELIX
CCW-SPIRAL

10

RIGHT-HANDED HELIX
CW-SPIRAL

v1, 46          47

Y
↑
→X
Z

## FIG. 1C

vr2   vr1   vr3   vr4

10

19

SIDE
PORTION A

SIDE
PORTION B

Y
↑
→X
Z

## FIG. 1D

v1, 46

Y
↑
→X
Z

**Description**

BACKGROUND OF THE INVENTION

Field of the Invention

The claimed disclosure relates to a transducer array that measures an acoustic wave and, in particular, relates to a transducer array that measures an acoustic wave that is generated by a photoacoustic effect.

Description of the Related Art

A photoacoustic tomography (PAT) diagnostic apparatus utilizing the photoacoustic effect is known. In such an apparatus, an object is irradiated by an illumination light (near-infrared light) from an Nd:YAG laser pulse light source, and during the irradiation, an acoustic wave generated inside the object by the photoacoustic effect is received by a two-dimensional or a three-dimensional array transducer (transducer array) to generate and display an image.

PCT Japanese Translation Patent Publication No. 2001-507952 discloses a photoacoustic measurement apparatus provided with a transducer array in which a plurality of transducers are arranged in a supporting structure having a curved surface shape in order to increase accuracy in the separation and synthesis of acoustic wave signals propagated from the subject through each propagation path. Furthermore, the transducer disclosed in PCT Japanese Translation Patent Publication No. 2001-507952 is a piezoelectric transducer including a round receiving surface.

Owing to micro-electric-mechanical system technology, a transducer formed of a capacitive micromachined ultrasonic transducer (a CMUT) that has high density, high sensitivity, and wide reception band properties is known. The micro-electric-mechanical system technology is also referred to as Micro Electro Mechanical systems (MEMS) technology, and hereafter, in the present specification, the technology will be referred to as MEMS.

Since the CMUT element is formed through a semiconductor process, the CMUT element is cut out from a semiconductor wafer 18 using a dicing cut technique that is capable of cutting the semiconductor wafer 18 into a predetermined shape and size. Accordingly, in order to increase the yield of the semiconductor wafer, the outer edge shape of the CMUT element is not circular as in PCT Japanese Translation Patent Publication No. 2001-507952, but has a parallelogram shape including a square shape.

When mounting such transducers having a parallelogram-shaped receiving surface on a transducer array, in an arrangement in which one of the vertexes that define a receiving surface and one of the vertexes that define an adjacent receiving surface are proximate to each other, the mount density of the transducers decreases and the resolution of the image becomes disadvantageously limited. Furthermore, in an arrangement in which one of the vertexes defining the receiving surface and a side that defines the adjacent receiving surface are proximate to each other, the effective reception regions overlaps each other and crosstalk is disadvantageously generated, disadvantageously restricting the resolution of the image. Furthermore, in such a mode, when an area of a receiving surface of a single transducer is reduced in order to secure a mount density of the receiving surface, consequently, the effective reception region becomes limited and the receiving sensitivity of the transducer array decreases, making it disadvantageously difficult to obtain information of a deep portion inside the subject.

SUMMARY OF THE INVENTION

The present disclosure in its first aspect provides a transducer array as specified in claims 1 to 11.
The present disclosure in its second aspect provides an acoustic wave measurement apparatus as specified in claims 12 to 16.
The present disclosure in its third aspect provides a transducer array as specified in claims 17 to 20.
The present disclosure in its fourth aspect provides an acoustic wave measurement apparatus as specified in claims 21 to 24.
The present disclosure in its fifth aspect provides a transducer array as specified in claim 25.
The present disclosure in its sixth aspect provides a transducer array as specified in claim 26.
Further features of the present invention will become apparent from the following description of exemplary embodiments with reference to the attached drawings.

BRIEF DESCRIPTION OF THE DRAWINGS

Figs. 1A to 1D are diagrams related to the transducer array of a first exemplary embodiment of the claimed disclosure, in which Fig. 1A is an overhead view, Fig. 1B is a top view, Fig. 1C is a partially enlarged view, and Fig. 1D is a top view.
Fig. 2 is a block diagram of an acoustic wave measurement apparatus according to a second exemplary embodiment of the claimed disclosure that measures a photoacoustic wave.
Fig. 3 is a conceptual diagram illustrating a CMUT element formed on a semiconductor wafer.
Fig. 4 is a conceptual diagram illustrating a scanning trace of a support apex of the transducer array.
Fig. 5 is a cross-sectional view illustrating a cross section of the transducer array related to the first exemplary embodiment of the claimed disclosure.
Figs. 6A to 6D are cross-sectional views illustrating exemplary embodiments of supports.

Figs. 7A to 7D are plan views illustrating exemplary embodiments of the receiving surface.

## DESCRIPTION OF THE EMBODIMENTS

### Exemplary Embodiment

#### Acoustic wave measurement apparatus

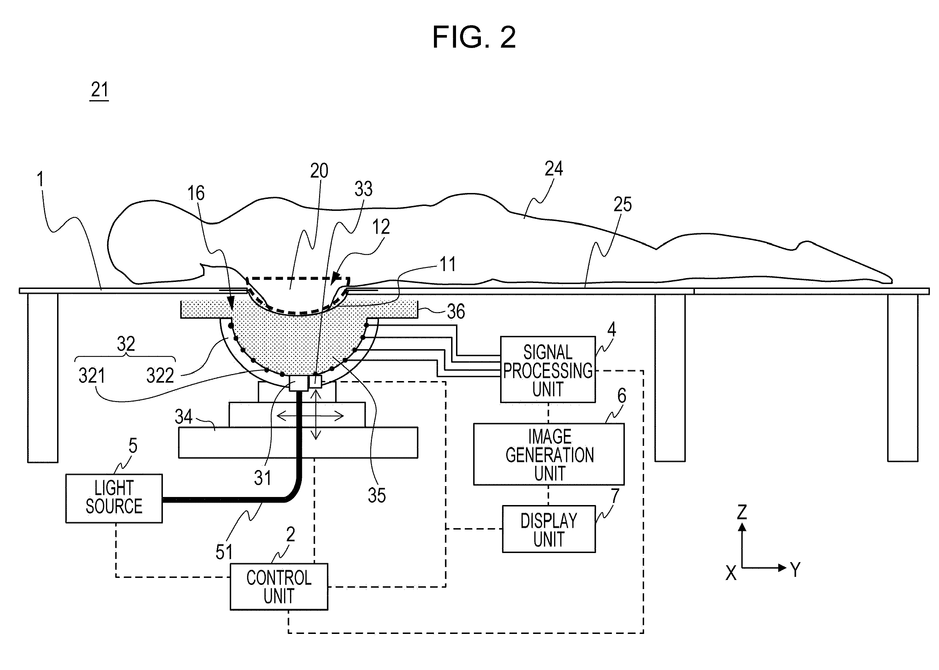

Fig. 2 is a block diagram illustrating a second exemplary embodiment in which a transducer array 32 of the claimed disclosure is applied to an acoustic wave measurement apparatus 21 that measures a photoacoustic wave. A supporting base 1 includes a supporting surface 25 that supports a subject 24 from below, and an insertion opening (opening) 12 that is provided in the supporting surface 25 and into which an object 20 is downwardly inserted. The object 20 is held on a holding member 11 provided in the supporting base 1. The object 20 might include breasts, four limbs, a head, and the like of the subject 24. The holding member 11 has a bowl-like so that the object 20 can be easily placed therein.

The transducer array 32 sends an acoustic wave to the object 20, and the acoustic wave reflected by the object 20 is received by the transducer array 32. Alternatively, the transducer array 32 receives, as an acoustic wave, a pressure elastic wave that is created as a result of a local and short time change, such as swelling or contraction, of the object 20 caused by the object 20 being irradiated with a pulsed light from a light irradiation unit (light irradiation means) 31 described later.

In the transducer array 32, receiving surfaces 10 included in transducers 321 are disposed so as to be oriented towards the object 20. The receiving surfaces 10 are connected to a vessel 36 and a support member 322 such that the receiving surfaces 10 are oriented towards the inside of the vessel 36; accordingly, the transducers 321 are capable of efficiently acquiring the acoustic wave propagating from the object 20.

A matching liquid 35 is filled and stored in the vessel 36 between the transducer array 32 and the holding member 11. The transducer array 32 is connected to the vessel 36 filled with the matching liquid 35. Furthermore, it can be said that the transducer array 32 constitutes a portion of the vessel 36 in which the matching liquid 35 is filled. The vessel 36 includes an opening portion 16 that is acoustically opened at a position that overlaps the insertion opening 12 so that the object 20 can be in acoustic contact with the matching liquid 35 stored in the vessel 36. The transducer array 32 is connected to the vessel 36 on a side that faces the opening portion 16.

Water, an acoustic impedance of which is closer to the acoustic impedance of the tissue of the subject than the acoustic impedance of air, is used in the matching liquid 35. Other than water, gel or the like may be used as the matching liquid 35.

The transducer array 32 includes a camera 33 for observing the state in which the object 20 is held by the holding member 11, the transducers 321, and a vessel scanning unit 34 that moves the vessel 36 in two dimensions along a horizontal plane (an XY plane).

The vessel scanning unit 34 moves the vessel 36 relative to the insertion opening 12 to perform two-dimensional scanning of an effective reception region of the transducer array 32 along a horizontal plane such that the object 20 and the effective reception region overlap each other. It can be said that in the present exemplary embodiment, in a state in which the light irradiation area (not shown) irradiated by light from the light irradiation unit 31 is overlapped with the object 20, the light irradiation area is capable of performing two-dimensional scanning along the horizontal plane.

Note that an XY vessel scanning unit, which is a combination of a linear guide, a feed screw mechanism, and a motor, and the like (all not shown), is also included in the vessel scanning unit 34 of the claimed disclosure.

A signal processing unit 4 performs an AD conversion and performs signal processing on digital data (acoustic data). An image generation unit 6 generates a two-dimensional or three-dimensional acoustic image using the acoustic data. The display unit 7 displays the generated acoustic image and the image acquired with the camera 33. A control unit 2 integrally controls at least the XY vessel scanning unit, the light source 5 and the signal processing unit 4.

### Holding member

The holding member 11 has a maintaining function that holds the shape of the object 20 to a predetermined shape during measurement, and has an acoustic impedance that does not hinder the propagation of the acoustic wave reaching the transducer array 32 from the object 20. While the holding member 11 is selected according to the form of the object 20, when the object 20 is a breast, a bowl-like curved surface support is used. Regarding the holding member 11, a plurality of replaceable supports that have sizes and curvatures according to the sizes and shapes of the objects are prepared, or an elastomer and the like that has flexibility is applied.

Note that in order to facilitate transmission of the acoustic wave, a thickness as thin as 0.05 mm to 1.0 mm is applied to the holding member 11. Furthermore, in the present exemplary embodiment, the holding member 11 is a light transmitting member that transmits pulsed light irradiated from the light irradiation unit 31. In a case of an acoustic wave measurement apparatus (not shown) in which the light irradiation unit 31, a light guiding member 51, and a light source 5 are removed from the present exemplary embodiment, the light transmitting property of the holding member 11 is not necessarily required.

Since the holding member 11 maintains the photographing position, the holding member 11 is, desirably, configured of a member that has strength that can endure the load of the object 20, and polyethylene terephthalate (PET), for example, is applied.

Light source

The light source 5 generates a photoacoustic wave inside the object 20 by having the object 20 be irradiated by pulsed light. When the object 20 is a tissue, the light source 5 emits a light with a specific wavelength that is absorbed by a specific component among the components constituting the object 20. The light source 5 may be provided together with the transducer array 32 in an integrated manner; however, as in the present exemplary embodiment, the light source 5 may be separated from the transducer array 32 through the light guiding member 51 and may be optically connected.

The light source 5 is desirably a pulse light source that is capable of generating, as an irradiation light, a pulsed light that has a pulse width of a few nanoseconds to a few hundred micro seconds; however, with an intention to generate a photoacoustic wave in an efficient manner, the present exemplary embodiment is configured so that a pulse width between 10 to 100 nanosecond can be adjusted.

Since a high output can be obtained, laser is desirable for the light source 5; however, a light emitting diode or the like may be used instead of the laser. As for the laser, various lasers such as a solid-state laser, a gas laser, a fiber laser, a dye laser, and a semiconductor laser can be used. The irradiation timing, the waveform, and the intensity are controlled with a light source controller (not shown). In the claimed disclosure, the wavelength of the light source that is used is desirably an infrared wavelength in which the light propagates to the inside of the object 20. A center wavelength of light emitted of the light source 5 in the present exemplary embodiment is 500 nm or larger to 1200 nm or smaller, that is, the light is a near-infrared light.

Light guiding member

Light that has been emitted from the light source 5 is guided to the object 20 while being processed into a desired light distribution shape with the light guiding member 51. The light guiding member 51 includes an articulating arm in which optical fiber and a lens-barrel with a mirror are built therein.

The light guiding member 51 includes, other than the above, a mirror that reflects light, a lens that collects, magnifies, and changes the shape of light, and a diffusing plate that diffuses light, for example. Transducer array

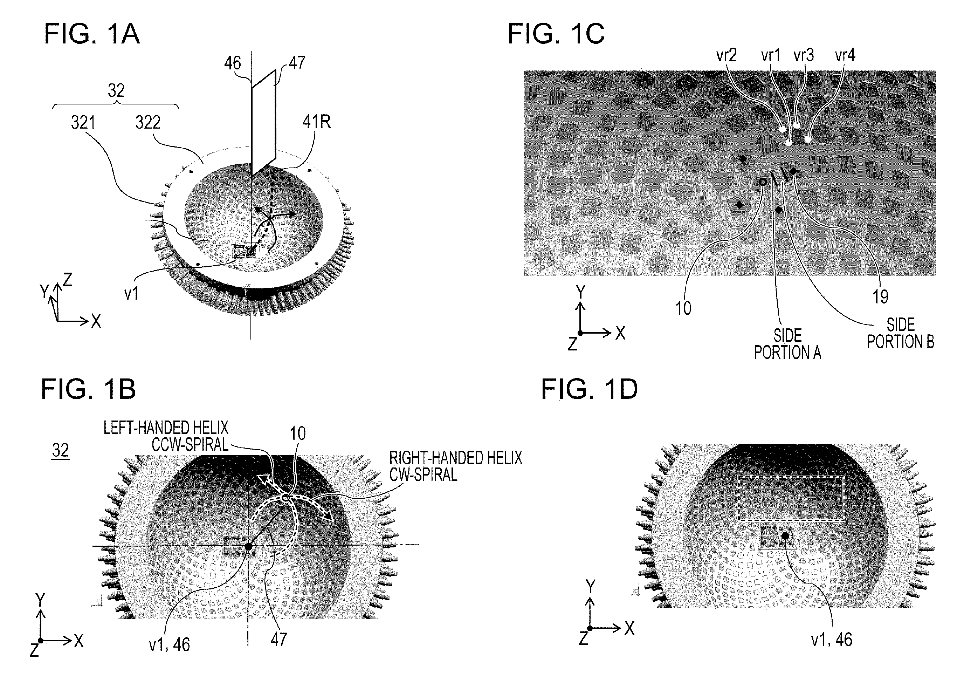

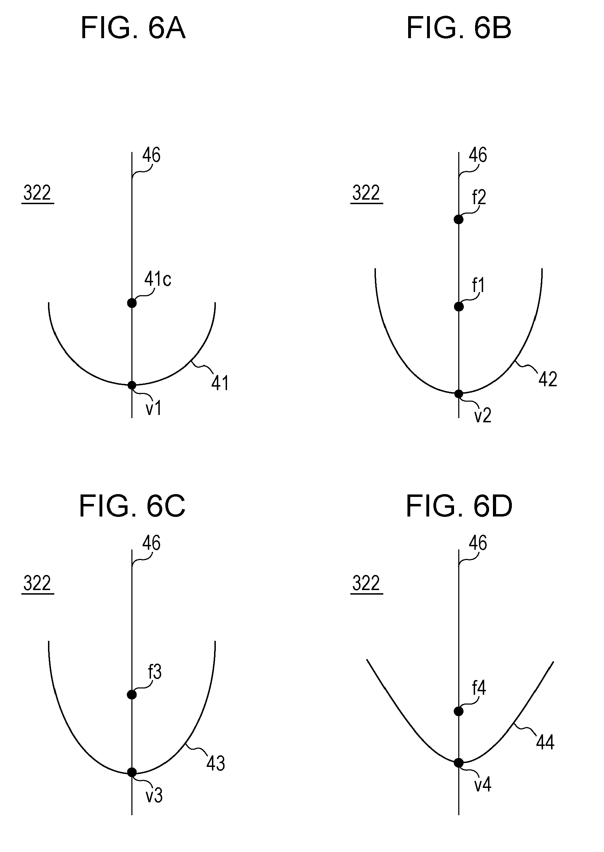

Figs. 1A to 1D are diagrams related to the transducer array 32 of a first exemplary embodiment of the claimed disclosure, in which Fig. 1A is an overhead view, Figs. 1B and 1D are top views, and Fig. 1C is a partially enlarged view. Furthermore, Figs. 6A to 6D are cross-sectional views of the transducer array 32 related to the first exemplary embodiment of the claimed disclosure.

As illustrated in Figs. 6A to 6D, the transducer array 32 includes the bowl-like support member 322 provided with a plurality of acoustically opened openings 40, the plurality of transducers 321 that are each connected to a corresponding one of the plurality of openings 40 and that transduces an acoustic wave into an electric signal. In other words, the transducer array 32 includes a bowl-like support member 322, an inner surface of which is defined by a quadric surface of revolution, and the plurality of transducers 321, each of the receiving surfaces 10 thereof being connected thereto so as to be oriented towards the inner side of the bowl-like inner surface, that transduce an acoustic wave into an electric signal. The quadric surface of revolution will be described later. The support member can be changed to a various modified embodiments including an integrated bowl-like structure (not shown) having a plurality of sub-support members each of which is secured to the corresponding one of the plurality of transducers.

Note that since the transducer array 32 of the present exemplary embodiment is, as illustrated in Figs. 1B and 1D, applied to the acoustic wave measurement apparatus 21 that measures a photoacoustic wave, the transducer array 32 includes the camera 33 that observes the light irradiation unit 31 connected through the light source 5 and the light guiding member 51, the transducers 321, and the held state of the object 20.

The claimed disclosure is an array of a plurality of receiving surfaces in the transducer array in which the shape of the inner surface is defined by a quadric surface of revolution, which will be described later.

Transducer

The transducers 321 each detect an acoustic wave and transduce the acoustic wave into an electric signal that is an analog signal. Any element (or transducing element) that is capable of detecting an acoustic wave, such as a transducing element utilizing a piezoelectric phenomenon, a transducing element utilizing optical resonance, or a transducing element utilizing change in capacity, may be used.

In the present exemplary embodiment, by disposing the plurality of transducers 321 in the support member 322 with a gap between each other, the transducer array 32 including a multidimensional array of transducers is formed. Using such a multidimensional array of elements enables simultaneous detection of acoustic waves at a plurality of locations, and the detection time can be shortened.

As one of the transducers, a capacitive micromachined ultrasonic transducer is being developed that is fabricated using a micromachining process. A capacitive micromachined ultrasonic transducer includes a cell including a supported vibrating membrane having a gap between a lower electrode, and an upper electrode that is disposed on a surface of the vibrating membrane. Such a capacitive micromachined ultrasonic transducer is referred to as a capacitive micromachined ultrasonic transducer (CMUT). A CMUT is capable of forming a receiving surface including a few ten to a few thousand capacitive cells in a few square millimeters and employs a light-weight vibrating membrane; accordingly, a CMUT is capable of securing a wide receiving band.

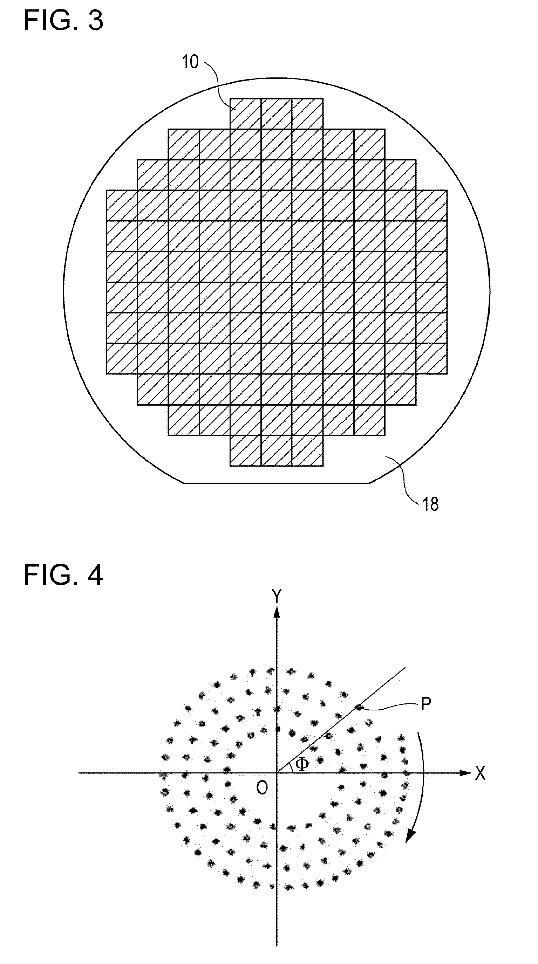

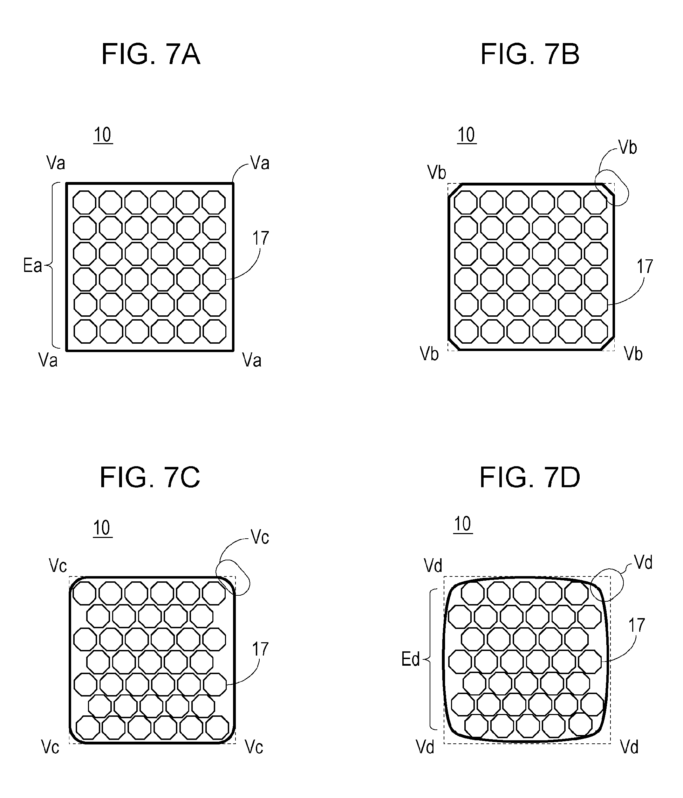

Fig. 3 is a schematic diagram of a CMUT element array formed on a semiconductor wafer 18. Furthermore, Figs. 7A to 7D are schematic diagrams illustrating exemplary embodiments of the receiving surfaces 10.

CMUT elements are formed from a semiconductor wafer 18 using a dicing cut technique such that an aggregate area of each vibrating membrane (membrane) is cut into a unit cell. The unit cell becomes a minimum unit of a channel reading out an electric signal based on an acoustic wave signal received by the transducer 321. In performing the dicing cut technique, cutting in a parallel manner is repeated at predetermined spatial intervals to cut out the required device areas. The CMUT elements including the cut out receiving surfaces 10 can be made to have a predetermined uniform shape and size.

Accordingly, as illustrated in Figs. 3 and 7A, the outer edges of the receiving surfaces 10 of the cut out CMUT elements have a substantially parallelogram shape including four vertexes. With the intent of reducing the orientation dependence of each transducer 321 when mounted on the transducer array 32, that is, from an aspect of symmetry, the CMUT element is square in the present exemplary embodiment.

Note that as illustrated in Fig. 7A, the receiving surface 10 of the present exemplary embodiment includes 6 x 6 = 36 unit cells of the CMUT element. The CMUT element sets the capacitance and includes an anode and cathode pair (not shown) for reading the capacitance change out to the exterior. The vibrating membrane (membrane) that receives the acoustic wave is acoustically bonded to either one of the anode and cathode pair. Accordingly, the receiving surface 10 of the CMUT element can be shaped as a vibrating membrane that is acoustically bonded to either one of the anode and cathode pair.

In the receiving surface 10 illustrated in Fig. 7A, the four vertexes Va are each right angled, and a side portion Ea forms a linear square. Furthermore, the receiving surface 10 of the present exemplary embodiment includes unit cells 17 arranged in a matrix.

Meanwhile, the receiving surface 10 illustrated in Fig. 7B is different from the receiving surface 10 illustrated in Fig. 7A in that the four vertexes Vb are each tapered at 45 degrees with respect to the side portions. In the present exemplary embodiment, a length of the tapered portion is desirably 14% or smaller than a side of a circumscribed square, which is a smallest parallelogram shape that inscribes the receiving surface 10 at the outside of the receiving surfaces 10.

Furthermore, the receiving surface 10 illustrated in Fig. 7C is different from the receiving surfaces 10 illustrated in Figs. 7A and 7B in that each of the four vertexes Vb is rounded into a quadrant and that unit cells 17 are arranged in a staggered manner. In the staggered array of the present exemplary embodiment, the phase of the unit cells 17 between each line is shifted 180 degrees.

Furthermore, the receiving surface 10 illustrated in Fig. 7D is different from the receiving surface 10 illustrated in Fig. 7C in that the side portions Ed are defined as bent lines.

As described above, not only a geometrically perfect square illustrated in Fig. 7A, each receiving surface 10 may be substantially square in which the adjacent side portions having the same length are practically orthogonal to each other. In other words, in the present specification, each receiving surface 10 do not have to, in a geometrically strict manner, have a parallelogram shape and as illustrated in Figs. 7B to 7D, the receiving surface 10 includes a substantially parallelogram shape that is defined by a semiconductor cutting process, such as dice cut, and a simple treatment of the peripheral portion. Note that the tapering and the rounding of the vertexes Vb and Vc of the receiving surfaces 10 illustrated in Figs. 7B and 7C are intended to reduce chipping and breakage of the vertex from occurring during handling after the cutting.

Note that the transducer that can be applied to the transducer array of the claimed disclosure includes, other than CMUT, a piezoelectric acoustic wave transducer (a piezoelectric ultrasonic transducer).

Support

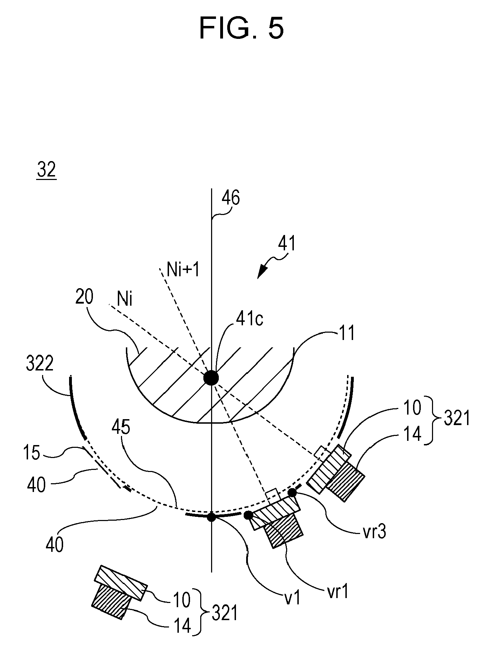

Referring to Figs. 1 and 5, description of the support member 322 will be given next. The support member 322 supports the plurality of transducers 321 along the openings provided in the support member 322. The present exemplary embodiment is a transducer array 32 in which the plurality of transducers 321 are each connected to the corresponding one of the plurality of openings 40 provided in the hemispherical support member 322.

The plurality of transducers 321 are connected to the support member 322 so that the receiving surfaces of the transducers 321 are oriented towards the inner side of the bowl-like bent surface. From the aspect of symmetry, the bent surface is desirably a quadric surface of revolution. Generally, a quadric surface of revolution includes, other than a sphere, a hemisphere, an ellipsoid, a paraboloid of revolution, and a hyperboloid of revolution, a cone, a column, a parabola pillar, a hyperbolic pillar, an elliptic cylinder, a sinusoidal column, and a parabola pillar. On the other hand, the quadric surface of revolution of the claimed disclosure includes a sphere, a hemisphere, an ellipsoid, a paraboloid of revolution, and a hyperboloid of revolution.

In other words, the quadric surface of revolution of the claimed disclosure is a mode that satisfies an "undevelopable surface requirement" and an "isocenter requirement".

The undevelopable surface requirement is a requirement in which a collimated problem occurs between adjacent receiving surfaces when receiving surfaces each having a parallelogram shape are arranged. The undevelopable surface requirement corresponds to the problem to be solved by the claimed disclosure. Note that a developable surface refers to a bent surface that is formed only by deformation without having to swell or contract a development view depicted on a flat surface, and the undevelopable surface refers to a bent surface that is not formed without having to partially swell or contract a development view depicted on a flat surface.

Furthermore, the "isocenter requirement" is a condition for forming a high-sensitivity region formed when effective reception regions of a transducer array formed of a plurality of transducers overlap each other at a specific single point. The isocenter requirement is a requirement that corresponds to the precondition of the claimed disclosure. Such a specific single point is referred to as an isocenter and when the inner surface of the transducer array is a hemisphere, the center of curvature of the hemisphere coincides with the isocenter. The quadric surfaces of revolution illustrated in Figs. 6A to 6D each have an isocenter 41c, f1, f3 and f4.

As illustrated in Fig. 5, the inner surface of the support member 322 is defined by a quadric surface of revolution 45 that is formed by rotation of an imaginary quadratic curve 41 about an imaginary rotation axis 46. In the present exemplary embodiment, the imaginary quadratic curve is a circle and the inner surface of the support member 322 is defined by a hemispheric shape. By having the inner surface of the support member 322 be a quadric surface of revolution, the acoustic wave propagated from the object that is disposed at a position that overlaps a rotation axis 46 can be received without being affected by reflection, attenuation, and the like caused by other structures around the transducer array 32 in which the inner surface thereof is defined by the quadric surface of revolution. The quadric surface of revolution 45 of the present exemplary embodiment is a hemisphere.

Note that in Fig. 5, the matching liquid disposed between the object 20 and the transducer array 32 is omitted.

Without loss of generality, it can be understood that the effective reception regions of the receiving surfaces 10 of the transducers 321 are around the normal lines Ni, Ni+1 in the drawing. As illustrated in Fig. 5, the transducer 321 is connected to the support member 322 so that the normal line Ni of the receiving surface 10 is oriented towards the inner side of the quadric surface of revolution 45 that surrounds the portion around the rotation axis 46. Note that in the present exemplary embodiment, a normal line N of one of the receiving surfaces 10 overlaps each other at the isocenter 41c of the hemisphere of the quadric surface of revolution 45.

Note that the support member 322 includes the opening portion 16 in a direction in which the object 20 is disposed. Furthermore, the support member 322 includes a support apex v1 on the side that is most far away from the opening portion 16. Furthermore, the support member 322 includes the plurality of openings 40, and the receiving surfaces 10 are each substantially parallel to a corresponding tangent plane 15 of the quadric surface of revolution intersecting the opening 40. In the present exemplary embodiment, the angle formed between each receiving surface 10 and the corresponding tangent plane 15 is within a $\pm$ 3 degrees range.

Figs. 6A to 6D illustrates, as a third exemplary embodiment, modes in which the imaginary quadratic curve that is the basis of the quadric surface of revolution is either a circle 41, an ellipse 42, a parabola 43, or a hyperbola 44.

As illustrated in Fig. 6A, when the imaginary quadratic curve is the circle 41, the imaginary rotation axis 46 is set so as to coincide with the diameter of the circle 41. Furthermore, as illustrated in Fig. 6B, when the imaginary quadratic curve is the ellipse 42, the imaginary rotation axis 46 is set to the major axis or the minor axis of the ellipse. In the present exemplary embodiment, the imaginary quadratic curve coincides with the major axis of the ellipse 42. Furthermore, as illustrated in Fig. 6C, when the imaginary quadratic curve is the parabola 43, the imaginary rotation axis 46 is set to an imaginary axis connecting a focus f3 and a vertex v3 of the parabola 43. Furthermore, as illustrated in Fig. 6D, when the imaginary quadratic curve is the hyperbola 44, the imaginary rotation axis 46 is set to an imaginary shaft connecting a focus f4 and a vertex v4 of the hyperbola 44.

Note that as illustrated in Figs. 1A and 5, the openings 40 are open in the support member 322 so that the openings 40 is shaped so as to conform with the receiving surfaces 10. In the present exemplary embodiment, each opening 40 is substantially square in which the adjacent side portions with the same length are practically orthogonal to each other.

Arrangement of receiving surface on quadric surface of revolution

The arrangement of the receiving surfaces on the quadric surface of revolution, which is a feature of the claimed disclosure will be described next with reference to Figs. 1A to 1D.

Referring to Figs. 1A to 1D and Fig. 5, a transducer array 32 related to the first exemplary embodiment is illustrated, the transducer array 32 including the support member 322 provided with the plurality of square openings 40, and the plurality of transducers 321 each including, corresponding to the opening 40, the receiving surface 10 and a transducing element 14.

In order to understand the arrangement of the receiving surfaces 10 on the quadric surface of revolution, one of the receiving surface, on which focus will be given, will be considered as a representative example. Such generalization may be applied to almost all of the portions in the arrangement of the receiving surface except for a singular point, such as a peripheral portion in the arrangement of the receiving surface.

Fig. 1C is a partial enlarged view of a rectangular shaped portion depicted by a broken line in Fig. 1D for describing the arrangement of the receiving surface in detail. Fig. 5 is a schematic cross-sectional view related to the first exemplary embodiment illustrating the transducers 321 connected to the openings 40 of the support member 322 and a transducer 321 in a non-connected state.

The receiving surfaces 10 illustrated in Figs. 1C, 1D, and 5 have a substantially parallelogram shape including four vertexes. One of the vertex (vr1) is closer to the imaginary rotation axis 46 than the other three vertexes (vr2, vr3, vr4). In other words, one of the vertex (vr1) is closer to the imaginary rotation axis 46 than the other three vertexes (vr2, vr3, vr4). The imaginary rotation axis 46 passes through the support apex v1 of the support member 322 and is disposed parallel to a Z shaft.

In the present exemplary embodiment, as illustrated in Fig. 7A, the receiving surface 10 includes four side portions, each of which is positioned between two adjacent vertexes. In Fig. 1C, the receiving surface 10 includes a side portion A, on which focus is given. Meanwhile, an adjacent receiving surface 19 that is one of four adjacent receiving surfaces (mark ♦) surrounding the receiving surface 10 (mark ○), on which focus is given, includes a side portion B on the side of the receiving surface 10, on which focus is given. The four adjacent receiving surfaces are 1st to 4±h proximal receiving surfaces. The receiving surface 10 and the adjacent receiving surface 19 are adjacent to each other with a substantially parallel gap portion in between. With the above, overlapping of the effective reception regions of the receiving surface 10 and the adjacent receiving surface is suppressed and crosstalk between the adjacent receiving surfaces can be suppressed. The gap portion is a portion of the support member 322 positioned between the side portion A and the side portion B. The side portion A and the side portion B that define the gap portion are substantially parallel to each other forming an angle from 0 degrees or larger to 10 degrees or smaller.

By disposing the receiving surface in the above manner, the receiving surface 10 is disposed so as to be surrounded by the four adjacent receiving surfaces that are adjacent to the receiving surface 10 while being spaced apart at a distance that is smaller than the fifth distance among the distances between the other receiving surfaces. By shortening the distance between the adjacent receiving surface and the receiving surface 10, on which focus is given, the mount density of the transducers 321 can be increased and crosstalk between the effective reception regions of the adjacent transducers 321 can be suppressed.

Furthermore, by disposing the substantially square shaped receiving surfaces 10 in the above parallel arranged manner, the receiving surfaces 10 are capable of forming a spiral array on the quadric surface of revolution at the corresponding positions of the openings 40 such that crosstalk between the effective reception regions of the adjacent transducers 321 can be suppressed and the mount density of the transducers 321 in the transducer array 32 can be increased.

In an overhead view of Fig. 1A, the receiving surfaces 10 are arranged in a spiral manner with respect to the support member 322, the inner surface of which is defined by a hemisphere that is obtained by rotating a quadrant about the imaginary rotation axis 46.

The spiral arrangement of the receiving surfaces 10 is formed by a plurality of points of intersection between two types of spiral arrays that extend along the inner surface of the support member 322 from the support apex v1 side towards the opening portion of the support member 322 (in Fig. 1A, in the Z direction).

As illustrated in Fig. 1A, one of the spiral arrays is an array that extends along a right-handed helix that crosses an imaginary quadratic curve 41R, and the other spiral array is an array that extends along a left-handed helix that crosses the imaginary quadratic curve 41R. As described above, while securing the parallelism of the gaps between the receiving surfaces 10, the number of arrays of the transducers 321 is increased from the support apex v1 towards a rim of the support member 322. The number of arrays of the transducer 321 may be set to increase on the basis of the Fibonacci numbers, or the Tribonacci numbers that is a modification of the Fibonacci numbers.

Note that the imaginary quadratic curve 41R coincides with an imaginary quadratic curve that is defined by intersections between an imaginary plane 47 including the imaginary rotation axis 46 and the support member 322, and in the present exemplary embodiment, is a quadrant. Furthermore, it may be said that the imaginary quadratic curve 41R illustrated in Fig. 1A is one of the group of imaginary quadratic curves, which is the basis of the quadric surface of revolution 45 (Fig. 5) defining the inner surface of the support member 322.

As described above, by arranging a plurality of receiving surfaces 10, which have a substantially parallelogram shape on a plane, on the support member 322 including the inner surface formed of the quadric surface of revolution 45, it is possible to achieve high density mounting of the transducers 321 on the support member 322.

The present exemplary embodiment may be a mode described in the following manner.

The transducer array 32 includes the bowl-like support member 322 for supporting the plurality of transducers 321, in which the transducers 321 each include the receiving surfaces 10 that have a substantially parallelogram shape and that receives an acoustic wave.

Furthermore, the plurality of receiving surfaces 10 included in a predetermined area of the support member 322 are arranged on a grid formed by points of intersection between a plurality of imaginary right-handed spirals CW (clock wise) and a plurality of imaginary left-handed spirals CCW (counter clock wise), which cross the imaginary right-handed spirals CW, that extend along the inner surface from the side on which the support apex v1 of the bowl-like inner surface of the support member 322.

Note that the predetermined area described above is an optional area in which the receiving surfaces 10 are arranged in at least two directions that are not parallel to each other and, for example, includes a rectangular area depicted by a broken line in Fig. 1D.

Accordingly, the predetermined area described above is an area including at least four receiving surfaces 10 that are adjacent to each other in at least two array directions.

Note that as for a non-bowl-like support that is not the target of the claimed disclosure, there are non-quadric surfaces, such as a column and elliptic cylinder. For example, in a columnar support in which the inner surface is defined with a column, when receiving surfaces with a substantially parallelogram shape are arranged, there is no constraint in the geometries of the vertexes and the imaginary rotation axis such that a mode in which the receiving surfaces are mounted in a close-packed manner can be achieved without any spiral array.

Scanning trajectory of transducer array

A trajectory of a transducer array in an ultrasonic wave measurement apparatus according to the second exemplary embodiment will be described with reference to Fig. 4. The scanning trajectory of the present exemplary embodiment is a spiral trajectory. The spiral trajectory is a scanning trajectory in which the transducer array is moved so that the coordinates in a radial direction changes so as to either increase or decrease with respect to the rotation center.

Fig. 4 is a diagram schematically illustrating an example of a motion of the spiral trajectory with the support apex v1 of the transducer array as a representative example. The point O in Fig. 4 represents a position of the support apex v1 before the spiral scanning and corresponds to a point of origin. Furthermore, black dots in Fig. 4 indicates the position of the support apex v1 during photoacoustic wave measurement. The transducer array moves while sequentially tracing the positions of the black dots. Point P is a single point on the trajectory of the moving transducer array. When the position coordinate (x, y) of point P is expressed with a polar coordinate system, the following equations (1) are obtained.

$$x = r(t)\cos\varphi$$

$$y = r(t)\sin\varphi \qquad \cdots\cdots(1)$$

Note that r(t) is a coordinate (moving radius) in the radial direction, and $\varphi$ is an angle formed by the X axis and a line extending from the point of coordinate origin towards point P. In the present exemplary embodiment, the transducer array is moved so that the coordinate of the transducer array on the moving trajectory in the radial direction, that is, r(t), changes so as to either increase or decrease. Furthermore, it is desirable that the vessel scanning unit moves the transducer array so that the velocity of the moving trajectory in a tangential direction is constant.

Typically, the detection timing of the photoacoustic wave is determined by the repetition frequency of the pulsed light emitted from the light source. For example, when a light source having a repetition frequency of 10 Hz is used, the photoacoustic wave can be generated once in 0.1 seconds. Accordingly, when the velocity in the tangential direction is uniform, and when it is assumed that detection of the photoacoustic wave is performed each 0.1 seconds, it will be possible to uniformly sample the space.

Furthermore, considering the acceleration towards the point of origin, it is desirable that the vessel scanning unit moves the transducer array from the outside of the moving plane. In other words, when the acceleration is large at the initial stage, shaking of the entire apparatus becomes large, and there are cases in which the shaking affects the measurement. Accordingly, shaking of the vessel can be reduced by starting to move from the outer periphery where the acceleration towards the point of origin is small towards the inner periphery.

Furthermore, it is desirable that the vessel scanning unit performs continuous motion in which motion is performed continuously rather than using a step and repeat method in which moving and stopping is repeated. With the above, the period of time in which the vessel is scanned can be shortened. Furthermore, since the change in acceleration during the motion is small, surface waviness of the matching liquid stored in the vessel can be reduced.

The claimed disclosure is capable of providing a transducer array that acquires an acoustic wave image that achieves both resolution and sensitivity when applied in measuring an acoustic wave. Furthermore, the claimed disclosure is capable of providing an acoustic wave measurement apparatus that exerts excellent quality in taking images.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions. For example, the present invention can be adapted for a hand-held type probe (not shown), a photo-acoustic wave detection system for a various parts of the human body.

**Claims**

1. A transducer array (32) comprising:

   a plurality of transducers (321) each of which is configured to receive an acoustic wave and to transduce the received acoustic wave into an electric signal; and
   a support member (322) supporting the plurality of transducers, and having an inner surface which is defined by a quadric surface of revolution (45) that is formed according to an imaginary quadratic curve (41, 42, 42, 44) rotating about an imaginary rotation axis (46),

   wherein each transducer includes

   a receiving surface (10) having a substantially parallelogram shape defined by four vertexes (vr1, vr2, vr3, vr4), the receiving surface being connected to the support member so as to be oriented towards the inner surface of the support, and
   a transducing element (14) configured to transduce an acoustic wave, received by the transducer via the receiving surface, into an electric signal,

   wherein the receiving surface is arranged in the transducer array such that any one of the four vertexes is closer to the imaginary rotation axis than the other three vertexes.

2. The transducer array according to claim 1,
   wherein in the case that the imaginary quadratic curve is a circle, the imaginary rotation axis is a diameter of the circle, or
   wherein in the case that the imaginary quadratic curve is an ellipse, the imaginary rotation axis is a major axis or a minor axis of the ellipse, or
   wherein in the case that the imaginary quadratic curve is a parabola, the imaginary rotation axis is an imaginary axis connecting a focus and a vertex of the parabola, or
   wherein in the case that the imaginary quadratic curve is a hyperbola, the imaginary rotation axis is an imaginary axis connecting a focus and a vertex of the hyperbola.

3. The transducer array according to claim 2,
   wherein the support member has an isocenter of the quadric surface of revolution, and

wherein the receiving surface is oriented to the isocenter such that a normal line of each one of the receiving surfaces over laps each other at the isocenter.

4. The transducer array according to any one of claims 1 to 3,
   wherein a plurality of openings that are acoustically opened are provided in the inner surface of the support member (322), and
   wherein the receiving surface is disposed in a corresponding one of the plurality of openings.

5. The transducer array according to claim 4,
   wherein the receiving surface is substantially parallel to a tangent plane of the quadric surface of revolution intersecting the corresponding opening.

6. The transducer array according to any one of claims 1 to 5,
   wherein the substantially parallelogram shape is substantially square in which adjacent side portions having equal length are practically orthogonal to each other.

7. The transducer array according to claim 4 or 5,
   wherein each opening of the plurality of openings is substantially square.

8. The transducer array according to any one of claims 1 to 7,
   wherein the receiving surface is surrounded by four adjacent receiving surfaces that are $1^{st}$ to $4^{\pm h}$ proximal receiving surfaces.

9. The transducer array according to claim 8,
   wherein the receiving surface and the adjacent receiving surfaces each include four side portions that are positioned between two adjacent vertexes among the four vertexes,
   wherein the adjacent receiving surfaces and the receiving surface are adjacent to each other with a substantially parallel gap between them, and
   wherein the gap portion forms an angle from 0 degrees to 10 degrees.

10. The transducer array according to any one of claims 1 to 9,
    wherein when assuming a point of intersection between the support member and the imaginary rotation axis as a support apex (v1, V2, V3 and V4), the plurality of receiving surfaces are arranged in the support member according to a left-handed spiral(CCW) and a right-handed spiral(CW) that intersect each other with the imaginary quadratic curve, defined by an intersection between an imaginary plane including the imaginary rotation axis and the support member, in between.

11. The transducer array according to any one of claims 1 to 10,
wherein the transducing element is further configured such that at least either of a capacitive micromachined ultrasonic transducer and a piezoelectric transducer (a piezoelectric ultrasonic transducer) is arranged two dimensionally.

12. An acoustic wave measurement apparatus (21), comprising:

a transducer array (32) according to any one of claims 1 to 11; and
a vessel (36) in which a matching liquid (35) is stored,

wherein the vessel includes an acoustically opened opening portion (16) that enables an object (20) to be in acoustic contact with the matching liquid, and
wherein the transducer array is connected to the vessel on a side that is opposite to the side on which the opening portion (16) is positioned.

13. The acoustic wave measurement apparatus according to claim 12,
the transducer array is connected to the vessel so that a receiving surface of a transducer is positioned to face the inside of the vessel.

14. The acoustic wave measurement apparatus according to claim 12 or 13, further comprising:

a supporting surface provided on an opening portion side of the vessel (36) and arranged to support a subject; and
an insertion opening (12) provided in the supporting surface such that the object can be inserted into the opening.

15. The acoustic wave measurement apparatus according to claim 14, further comprising:

a vessel scanning unit that moves the vessel in a relative manner with respect to the insertion opening provided in the supporting surface.

16. The acoustic wave measurement apparatus according to claim 14 or 15, wherein
the transducer array includes light irradiation means that emit near-infrared light or infrared light towards the insertion opening provided in the supporting surface.

17. A transducer array (32), comprising:

a plurality of transducers (321); and
a bowl-like support member (322) to support the plurality of transducers,

wherein each transducer includes a substantially parallelogram-shaped receiving surface (10) that receives an acoustic wave, and
wherein the plurality of transducers are supported by the support member such that a plurality of the receiving surfaces included in a predetermined area of the support member are arranged on a grid formed by points of intersection between a plurality of imaginary right-handed spirals (CW) and a plurality of imaginary left-handed spirals (CCW) which cross one another and extend along an inner surface of the support member from a side on which a center of the inner surface of the support member is positioned.

18. The transducer array according to claim 17,
wherein the receiving surface includes four vertexes that define a substantially parallelogram shape, and one of the four vertexes is closer to the center of the inner surface of the support member than the other three vertexes.

19. The transducer array according to claim 17 or 18,
wherein the substantially parallelogram shape is substantially square in which adjacent side portions having equal length are practically orthogonal to each other.

20. The transducer array according to any one of claims 17 to 19,
the predetermined area is an area including at least four receiving surfaces that are adjacent to each other in at least two array directions.

21. An acoustic wave measurement apparatus (21), comprising:

a transducer array (32) according to any one of claims 17 to 20; and
a vessel (36) in which a matching liquid (35) is stored,

wherein the vessel includes an acoustically opened opening portion (16) that enables an object (20) to be in acoustic contact with the matching liquid, and
wherein the transducer array is connected to the vessel on a side that is opposite to the side on which the opening portion (16) is positioned.

22. The acoustic wave measurement apparatus according to claim 21, further comprising:

a supporting surface provided on an opening portion side of the vessel and arranged to support a subject; and
an opening provided in the supporting surface such that the object can be inserted into the opening.

**23.** The acoustic wave measurement apparatus according to claim 22, further comprising:

a vessel scanning unit that moves the vessel in a relative manner with respect to the opening provided in the supporting surface.

**24.** The acoustic wave measurement apparatus according to claim 22 or 23, the transducer array includes light irradiation means that emit near-infrared light or infrared light towards the opening provided in the supporting surface.

**25.** A transducer array (32) comprising:

a plurality of transducers (321) each of which is configured to receive an acoustic wave and to transduce the received acoustic wave into an electric signal; and
a support member (322) supporting the plurality of transducers, and having an inner surface which is defined by a quadric surface of revolution (45),

wherein each transducer includes a receiving surface (10) being connected to the support member so as to be oriented towards the inner surface of the support, and
wherein the receiving surface is shaped a substantially square in which adjacent side portions having equal length are practically orthogonal to each other and defined by four vertexes (vr1, vr2, vr3, vr4).

**26.** A transducer array (32), comprising:

a plurality of transducers (321) each of which has a substantially square-shaped receiving surface (10); and
a bowl-like support member (322) to support the plurality of transducers,

wherein any one of pair of adjacent square-shaped receiving surface are arranged substantially in a parallel arranged manner in the bowl-like support member.

FIG. 1C

SIDE PORTION B

vr4
vr3
vr1
vr2
vr1

19

SIDE PORTION A

10

Y
Z
X

FIG. 1D

v1, 46

Y
Z
X

FIG. 1A

47
46

41R

32
322
321

v1

Y
Z
X

FIG. 1B

32

LEFT-HANDED HELIX
CCW-SPIRAL

RIGHT-HANDED HELIX
CW-SPIRAL

10

47

v1, 46

Y
Z
X

# FIG. 2

FIG. 3

FIG. 4

# FIG. 5

## FIG. 6A

## FIG. 6B

## FIG. 6C

## FIG. 6D

FIG. 7A

FIG. 7B

FIG. 7C

FIG. 7D

**EP 3 147 037 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001507952 PCT